# EUROPEAN PATENT APPLICATION

(11) **EP 4 537 865 A1**
(43) Date of publication of application: **16.04.2025**
(21) Application number: 23202424.0
(22) Date of filing: 09.10.2023
(51) Int. Cl.: A61M 3/02

(54) **AN IRRIGATION SYSTEM WITH IMPROVED FLOW CONTROL**

(71) Applicant: Wellspect AB, 431 21 Mölndal (SE)
(72) Inventor: D'Espindula, Gabriel, 418 71 Göteborg (SE); Wingren, Lukas, 583 29 Linköping (SE)
(74) Representative: Aldridge, Henry Alexander

(57) **Abstract**

An irrigation system comprises a reservoir for an irrigating liquid and a pressure sensor for measuring the pressure in the reservoir. A catheter is connected to the reservoir by tubing, providing a fluid communication path between the reservoir and the catheter. An electrically operated pump pumps air into the reservoir to indirectly pump irrigation liquid from the reservoir to the catheter, and an electrically operable valve is operable to continuously control the degree of openness of the fluid communication path between a fully closed state and a fully opened state. Further, a sensor estimates power consumption or speed of the electrically operated pump. A controller estimates an actual flow rate from the reservoir based on the estimation of power consumption and/or speed and continuously regulates the electrically operable valve based on the estimated actual flow rate, thereby adjusting the actual flow rate to the desired flow rate, obtained from a user interface,.

## Description

### TECHNICAL FIELD OF THE INVENTION

The present invention relates to an irrigation system, comprising a reservoir for an irrigation liquid, a catheter for arrangement in a user, and a control unit. The irrigation system is particularly intended for rectal, transanal and/or stomal irrigation, and is suitable for self-administration of an irrigation liquid.

### BACKGROUND OF THE INVENTION

Catheters and catheter systems are used for many types of medical procedures. For example, administrating an irrigation liquid is a common medical procedure whereby liquid is injected into a bodily cavity, such as into the rectum and lower intestine of a patient in order to induce bowel movement. The need for such a procedure typically arises in patients suffering from certain physical ailments, in which voluntary bowel control is impaired, or when the bowel needs to be cleaned before e.g. a coloscopy or a surgical operation. To this end, irrigation systems may be used e.g. by people suffering from spinal cord injuries, spina bifida or multiple sclerosis. For such users, irrigation may improve quality of life by preventing constipation, reducing time spent for bowel emptying procedures, reducing fecal incontinence, and by increasing independency in general.

Irrigation is nowadays often performed outside medical attendance premises, such as in the patient's home, and is also often performed by the patient himself, i.e. by self-administration. Thus, such irrigation systems need to be easy to control. Further, many of the users of irrigation systems have reduced dexterity, which makes simple operation even more important.

It is further of importance that the irrigation system is of a limited size, and portable. Portability of the irrigation system is important to disabled persons who are not hospitalized or bed-ridden if they are to live as normal a life as possible. This is particularly important if they travel away from their home, for instance, to someone else's home or if they stay in a hotel. In this situation, they need to be able to deal with their bowel function easily.

To this end, it has been proposed to use electrically operated irrigation systems, which simplifies the irrigation process. Electrically operated irrigation systems are e.g. disclosed in WO 2016/095929, US 2016/0114148, WO 2009/152568 and EP 2 679 261.

However, these systems are also subject to various problems, such as being somewhat complex and expensive. Further, the electrical systems of these known systems are often difficult to control with sufficient precision. For example, the desired flow rate varies widely between users. Sometimes a very limited flow rate is desired, whereas in other situations and for other users, a relatively high flow rate is desired. Thus, there is a need to control the flow rate with high precision and accuracy, and to provide a constant flow rate, with no or small variations during the irrigation process, at the set level. However, in known systems, this is difficult to achieve.

Some known irrigation systems, such as the one disclosed in EP 3 295 977, use indirect pumping, i.e. where a fluid such as air is pumped into a reservoir holding the irrigation liquid, and thereby increasing the pressure in the reservoir so that the irrigation liquid is pumped out from the reservoir. Such indirect pumping has many advantages compared to direct pumping, such as being easier to operate, less costly etc. However, it has been found that such irrigation systems using indirect pumping are particularly difficult to operate with adequate precision, e.g. in order to obtain a constant and exact flow rate.

Further, flow meters with adequate accuracy and precision are generally large and costly, making them unsuitable for use in irrigation systems, and in particular small and portable irrigation systems.

It may also be difficult to ensure that the irrigation system is always operated in a safe way, and unintended use of the system, either on purpose or by accident, may lead to discomfort or even hazard to the user/patient.

There is therefore a need for an irrigation device which can be used safely, easily and conveniently, and with improved controllability, for self-administration of the irrigation liquid, and which also preferably can be produced in a cost-efficient way.

### SUMMARY OF THE INVENTION

It is therefore an object to provide an irrigation system for medical use which at least alleviates the above-discussed drawbacks of the prior art. In particular, it is an object to provide an irrigation system for use in irrigation procedures, such as in rectal or transanal irrigation.

This and other objects are achieved with an irrigation system according to the appended claims.

According to an aspect, there is provided an irrigation system comprising:
a reservoir for an irrigating liquid;
a pressure sensor for measuring the pressure in the reservoir;
a catheter for arrangement in a user;
tubing providing a fluid communication path between said reservoir and said catheter;
an electrically operated pump for pumping air into the reservoir, thereby to indirectly pump irrigation liquid from the reservoir to the catheter through said tubing;
an electrically operable valve operable to continuously control the degree of openness of said fluid communication path between a fully closed state and a fully opened state;
a sensor for estimation of power consumption or speed of the electrically operated pump;
a controller arranged to estimate an actual flow rate of irrigation liquid from the reservoir to the catheter based on said estimation of the power consumption and/or speed, to obtain a desired flow rate from a user interface, and to continuously regulate the electrically operable valve based on the estimated actual flow rate, thereby adjusting the actual flow rate to said desired flow rate.

By "continuously regulating" is meant a regulation and control which is continuous over a certain period of time, such as during the time the pump is active, during the entire irrigation process, at all times, or the like.

The present invention is based on the realization that an actual flow rate of irrigation liquid from the reservoir to the catheter can be estimated based on the power consumption and/or speed of the electrically operated pump. Hereby, the actual flow rate can be estimated without any need for flow meters and the like. Such flow meters are generally relatively costly, thereby significantly adding to the overall costs of the system. A flow meter may also impede and obstruct the flow from the reservoir to the catheter, thereby reducing the performance of the system. It has now been found that an actual flow rate of irrigation liquid from the reservoir to the catheter can be estimated very accurately and reliably based on the power consumption and/or speed of the electrically operated pump, and in a very cost-efficient way. The controller may then control the output flow rate by controlling a degree of openness of the electrically operable valve. The valve is preferably controlled by a feedback control system, wherein the determined actual flow rate is continuously determined, and the electrically operable valve is controlled by the controller in real-time to adjust the degree of openness in accordance with the determined actual flow rate.

Hereby, a very precise and accurate control of the output flow rate can be obtained within a broad range of possible desired flow rates. For example, the flow rate can be controlled to be as low as 100 ml/min, or even lower, and as high as 700 ml/min or even higher.

With the present solution, the electric pump may e.g. be controlled to maintain an essentially constant pressure in the reservoir, regardless of the volume of irrigation liquid in the reservoir. The power and speed of the pump will vary depending on the outflow of irrigation liquid from the reservoir, in order to maintain the pressure at a constant level. When there is no outflow, i.e. when the valve is closed, and when the reservoir is closed, the pump may be idle. When there is an outflow, the pump needs to pump air into the reservoir to maintain the pressure level, and the greater the outflow rate, the greater the required speed and power consumption of the pump. The power and speed of the pump are consequently correlated to the flow rate of irrigation liquid out from the reservoir, and consequently also to the pumped volume of irrigation liquid, which is obtainable by integration of the flow rate over time. This correlation between the power consumption and speed of the pump can be determined by simple experiments.

In alternative embodiments, there may be a controlled limited outflow of air from the reservoir through a leak valve or the like. In such embodiments, the pump will be operated also when the electrically operated valve is closed, and where there is no outflow of irrigation liquid from the reservoir. The pump will then operate at an idle power consumption and speed to maintain the pressure constant in the reservoir, to compensate for the leakage. The difference between a determined power consumption and/or speed and the idle power consumption and/or speed, respectively, can then be used to determine an outflow rate and outflow volume of irrigation liquid from the reservoir when the electrically operated valve is opened. In such an embodiment, the pump will always remain active, and may hereby e.g. have a shorter reaction time when the electrically operated valve is opened.

A constant pressure in the reservoir may be obtained by a PID (proportional integral derivative) control, controlling the pump in dependence of the measured pressure in the reservoir. The control of the electrically operated valve may also be a PID control.

By the present solution, the actual flow rate of irrigation liquid out from the reservoir can be continuously and accurately determined and be used to control the actual flow rate to obtain a desired flow rate, which may be a predetermined flow rate or a flow rate set by the user.

The estimated flow rate of irrigation liquid can also be used to determine a total volume of irrigation liquid that has been pumped out from the reservoir, by integration of the flow rate over time. This total volume may also be used to control the irrigation system. For example, the irrigation system may be arranged to close the electrically operated valve when a target volume has been reached. The target volume may be a predetermined volume, or a volume set by the user.

The electrically operated pump is arranged for indirect pumping of the irrigation liquid. Hereby, the pump pumps a different fluid, such as air, into the reservoir, thereby increasing the pressure in the reservoir, and as a consequence forcing irrigation liquid out from the reservoir, for discharge through the probe/catheter.

An electrically operated pump is of great advantage in irrigation systems of this type, since it can be operated very easily, which is particularly advantageous for users with reduced dexterity. If the user lacks strength in their hands it may be easier for them to operate an electric pump rather than squeezing e.g. a foil-pump. The electrically operated pump can also easily be adjusted and customized for different types of use, for different types of users, etc.

It has been found by the present inventors that controlling the flow rate by conventional controlling of an electrically operated pump is often insufficient, and in particular when indirect pumping is used. It is often difficult to provide a flow rate with adequate precision, the flow rate is often unstable over time, and it is very difficult to obtain low flow rates, such as below 250 ml/min. The flow rate also often varies from time to time, or even during an irrigation process, due to changes in the contextual environment. For example, a higher flow rate is normally obtained if the reservoir is at a higher position in relation to the probe/catheter, such as being placed in a sink, on a table or the like, whereas a lower flow rate is normally obtained if the reservoir is at a lower position in relation to the probe, such as being placed on the floor. The counter pressure in the colon may also vary between different users, as well as over time for one and the same user.

However, by providing an estimate of an actual flow rate based on an estimate of power consumption and/or speed of the electrically operated pump, together with a measured pressure of the reservoir, enables a quick, accurate and reliable control of the flow rate out through the catheter. Hereby, by the control of the electrically operable valve, any desired flow rate can be obtained within a very large range, and with a very high precision and stability. Further, the control will adjust to contextual variations, such as variations in the counter pressure of the colon.

Still further, the new irrigation system facilitates operation, in particular for users having reduced dexterity. The whole irrigation procedure hereby becomes easier, faster and easier to control.

The control may also be realized in a very cost-effective manner, and without the need for costly and complicated sensors and the like.

Many types of electrically operable valves can be used in the above-discussed irrigation system. However, preferably the electrically operable valve is a clamping or pinch valve, providing a controllable clamping/pinching action on a tube leading between the electrical pump and the probe/catheter. For example, the valve may be of the type disclosed in US 2006/0114148 by the same applicant, said document hereby being incorporated in its entirety by reference.

In an embodiment, the electrically operable valve is a clamping or pinch valve, providing a controllable clamping/pinching action on the tubing.

Thus, the valve preferably comprises a clamping/pinching structure for a conduit or a tube. The terms "conduit" and "tube" are in the following used to indicate a structure enclosing a lumen and being made of an, at least somewhat, flexible material. The crosssectional shape is preferably circular, but other shapes, such as oval, may also be provided.

In one embodiment, the electrically operable valve comprises a movable arm that is connected to a constriction structure, said constriction structure being arranged opposite to an abutment, and with the tube extending between said constriction structure and said abutment, whereby movement of the moveable arm moves the constriction structure to control compression of the tube between said constriction structure and said abutment. The moveable arm may be movable in any suitable direction, such as being moveable longitudinally, e.g. by means of a linear motor, or be rotatable, e.g. by means of a servo motor. In a preferred embodiment, the moveable arm is rotatable, and comprising a cam shape engaging said constriction structure.

The electrically operated valve may, whenever activated, always be fully opened. Thus, the electrically operated valve may be operated intermittently between a fully closed state and a fully opened state. Thus, in an embodiment, the electrically operated valve is arranged to always be in one of two states, viz. a fully opened state and a fully closed state. Hereby, the valve could be operated intermittently, to switch between the binary states.

However, the valve need not be operated only at two states, and one or more intermediate states, having varying degrees of openness, could also be contemplated. In one embodiment, the degree of openness of the valve may be continuously changed between a fully opened and a fully closed state.

The valve is preferably arranged to remain in a set position without any power consumption. This can e.g. be realized by the above-discussed valve of the type disclosed in US 2006/0114148. Hereby, power is only needed to change the state and position of the valve. This lowers overall costs and size of the system, and e.g. makes it possible to use a smaller and less powerful battery.

In an embodiment, the electrically operable valve is operable to continuously control the degree of openness of said fluid communication path between a fully closed state and a fully opened state over a plurality of intermediate states between said fully closed and fully opened state, and preferably over essentially all possible intermediate states.

In an embodiment, the controller is further arranged to control a pumping speed of the electrically operated pump based on the measured pressure in the reservoir to provide an essentially constant pressure in the reservoir over a range of irrigation liquid filling levels. It has been found that by maintaining a constant pressure in the reservoir, independent on the irrigation liquid level in the reservoir, it is easier to provide an accurate estimate of the actual flow rate based on the power consumption and/or speed of the electrically operated pump. In such embodiments, the pressure determined by the pressure sensor is used to maintain the pressure in the reservoir at a constant level, and need not be considered in the estimation of the flow rate. A constant pressure also allows for a stable outgoing flow with minimal disturbances. Further, irrigation can be commenced quickly, since there is e.g. no need to find and establish a stable idle PWM (Pulse Width Modulation). There is also no need to know or determine the initial irrigation liquid volume.

However, alternatively, it is also feasible to allow the pressure in the reservoir to vary and base the determination of the flow rate also on the measured pressure in the reservoir.

Various types of sensors may be used to determine the power consumption and/or the speed of the electrically operated pump. In an embodiment the sensor for estimation of power consumption or speed of the electrically operated pump comprises a current and/or voltage sensor. In another embodiment, the sensor for estimation of power consumption or speed of the electrically operated pump comprises a speed sensor, and preferably a tachometer. The speed sensor may be realized in various ways, such as by use of a magnetic encoder, an optic encoder, etc., and the speed sensor may also detect speed by measuring vibrations or sound or the like.

In an embodiment, the controller is arranged to control the pumping speed of the electrically operated pump by controlling or measuring the voltage and/or current supplied to the electrically operated pump.

The irrigation system may further comprise an irrigation liquid level sensor for determining a current irrigation liquid level in said reservoir. The determined water level may be used as an additional input for determination of the flow rate, and thereby increase the accuracy of the flow rate estimation even further. In particular, this is of advantage in case the pressure in the reservoir is allowed to vary over time.

The electrically operated pump may be at least one of a peristaltic pump and a diaphragm pump. In an embodiment the electrically operated pump is a diaphragm pump. It has been found that diaphragm pumps are cost-efficient and reliable, and also relatively simple to analyze to determine a correlation between the supplied power and the resulting output flow of irrigation liquid from the reservoir.

The probe/catheter may be provided with an inflatable retention member. Inflation of the inflatable retention member may be effected by the same electrically operated pump being used for pumping the irrigation liquid. Alternatively, a second pump may be provided for inflation of the inflatable retention member. In this case, the second pump may also be an electrically operated pump, or alternatively be a manually operated pump, such as a bulb or a bellow pump.

In an embodiment, the irrigation system may further be provided with a property detection sensor to determine at least one property of the catheter, and to use the determined property to improve accuracy of the flow control even further.

The property detection sensor may comprise a Hall sensor, arranged to determine the presence and/or configuration of magnets in the catheter, or tubing connected to the catheter, the magnets being indicative of a size and/or type of the catheter. The Hall sensors may e.g. be arranged at, or in the vicinity of, where a catheter, or a tube connected to a catheter, is connected to another part of the system, such as a base unit. The Hall sensor may be used to simply determine whether magnets are present or not. However, the Hall sensors may also be used to determine specific patterns or combinations of magnets, specific number of magnets, specific field strengths of the magnets, etc. Hereby, it is possible to distinguish between two, three, four, or even more, types or properties of catheters.

Such determination of a catheter property based on Hall sensor detection is per se known from WO 2020/142185, said document hereby being incorporated in its entirety by reference.

Alternatively, detection of catheter property may occur in other ways, such as by provision of an RFID tag with information on the catheter, or tubing connected to the catheter, and an RFID reader connected to the controller. Such a solution is per se known from US 9 138 516, said document hereby being incorporated in its entirety by reference.

The irrigation system as discussed above and in the following may be used for rectal and/or stomal irrigation.

The system of the present invention is particularly useful for use in rectal or transanal irrigation system, as well as in stomal irrigation. However, the method/system of the present invention is also useable in other catheter systems useable for irrigation, and in particular irrigation of human body cavities.

In rectal or anal irrigation, an irrigation liquid is introduced into the rectum and lower intestine of a patient in order to induce bowel movement. The need for such a procedure typically arises in patients suffering from certain physical ailments in which voluntary bowel control is impaired or when the bowel needs to be cleaned before e.g. a coloscopy or a surgical operation. To this end, irrigation systems may be used e.g. by people suffering from spinal cord injuries, spina bifida or multiple sclerosis. For such users, irrigation may improve quality of life by preventing constipation, reducing time spent for bowel emptying procedures, reducing fecal incontinence, and by increasing independency and quality of life in general.

The irrigation system of the present invention is preferably portable, of limited size and relatively simple to use and control, also for user's having reduced dexterity. This makes it very well suited for self-administration, i.e. when irrigation is performed outside medical attendance premises, such as in the patient's home, and is performed by the patient himself. Further, portability of the irrigation system is of advantage for disabled persons who are not hospitalized or bed-ridden if they are to live as normal a life as possible. This is particularly important if they travel away from their home, for instance, to someone else's home or if they stay in a hotel. In this situation, they need to be able to deal with their bowel function easily.

Thus, the new irrigation system facilitates operation, in particular for users having reduced dexterity. The whole irrigation procedure hereby becomes easier, faster and easier to control, and at the same time the overall safety is increased due to the improved flow control.

The irrigation system may further comprise a control unit with a housing, said housing enclosing said electrically operated pump, said electrically operable valve and said controller. In an embodiment, the control unit may further be integrated with the reservoir. For example, the control unit housing may form a base or a platform on which the reservoir is arranged. The reservoir may be fixedly or releasably connected to the control unit housing.

The control unit further preferably comprises control elements for operation of the irrigation system. Additionally, or alternatively, such control elements may be provided on a remote control, wirelessly connected to the control unit. Preferably, the control unit and/or remote control comprises control elements for pumping of irrigation liquid, and for inflation and deflation of the inflatable retention member. The control unit and/or remote control may also comprise control elements operable to set a desired irrigation flow rate and/or desired inflation level.

The control unit and/or remote control may further comprise a display. The display may be used to assist in and confirm the selection of the desired flow rate and/or inflation level. Thus, the display may be used to show different available flow rates and/or inflation levels for the selection, and may also be used to show the selected rate/level, once the selection has been made.

Further, the display may be used to display information to the user about the progress of the irrigation procedure, such as volume that has been pumped, present flow rate, time elapsed from the start of the procedure, or estimated time left, etc. Further, the display may be used to guide the user about what choices in terms of settings and the like that are needed, the present function of the control elements, etc.

Still further, the display may be a touch screen, useable also for inputting data into the system. For example, the control elements may be realized as areas on the touch screen. If the control unit is connected wirelessly to a remote control or other remote unit, the display on this device may be used to display information. Thus a display on a remote control or other remote unit may be used to replace the display on the control unit, or to complement a display on the control unit.

By the use of a remote control, the control unit may e.g. be placed on the floor, or in any other resting position, and instead be operated through the remote control during irrigation. This facilitates handling of the irrigation system and affords the user an increased freedom in terms of how to use the system. The remote control may be a dedicated remote control, specifically arranged to control the irrigation system. However, the remote control may also be a common wireless device, capable of transmitting wireless control signals to a receiver in the control unit. In one preferred embodiment, the remote control is a mobile telephone, and preferably a smart phone. Additionally or alternatively, the remote control may be a laptop computer or a tablet computer. Hereby, a special application may be downloaded to the smart phone/laptop/tablet computer, providing a suitable interface for the device, and enabling it to send appropriate control signals to the control unit.

The control unit may further be arranged to transmit operation related data to a remote unit via wireless communication. For example, such data may be sent to a smart phone or the like. Hereby, the irrigation procedures may e.g. be logged over time, to facilitate follow-ups and also enabling a more adequate setting of parameters for forthcoming irrigations.

At least one, and preferably both of, the control element for controlling pumping of the irrigation liquid and the optional control element for pumping fluid for inflation of the inflatable member, preferably functions as a dead man's handle, thereby immediately returning to a deactivated state, in which the electrically operated pump is controlled not to pump, when manual operation of the control element is aborted. The control element(s) for inflation and deflation of the inflatable retention member may further be arranged as separate control elements. By means of this dead man's handle functionality it is ensured that pumping is immediately aborted when the control element is released. This means that the pumping action is stopped immediately when the control element is released, regardless of whether this release is intentional or by accident. For example, the pumping will stop immediately if the control element is accidentally dropped. Further, stopping by releasing is a very intuitive and quick operation method, which is both ergonomically favorable and fast.

The control elements may be operated by applying a predetermined condition to bring the control element into the activated state, and preferably at least one of depression, twisting, rotating, pulling and pushing. If a control button is used, the predetermined condition is preferably depression, so that the control button is activated by depressing it, and deactivated by releasing it. However, alternative types of control elements, such as rotatable knobs, switching levers and the like may also be used. An automatic return to the deactivated state when the predetermined condition ceases can e.g. be obtained by a spring, an elastic element or the like, operable to provide a counterforce to the force applied by the manual operation. The control elements, such as control buttons, may be arranged on the surface of the housing. The control elements may e.g. be realized as areas on a touch screen.

The control unit preferably comprises at least two control elements, such as control buttons, and preferably at least three control elements. Two, or preferably three, control elements enable a very easy manipulation of the control unit, and at the same time provides numerous input alternatives. It is further preferred that at least one of the control element(s) is a multi-purpose control element having different functions in different operation states. Hereby, the control elements can e.g. be assigned to control different functions during initiation/set-up and during operative use.

One or several control elements may also be arranged separated from the control unit, and may e.g. be connected with the control unit by means of wire, and thereby be physically connected to the electric pump etc. Alternatively, the control elements may be arranged on a remote control, which is wirelessly connected to the rest of the irrigation system. The remote control can e.g. be at least one of: a smart phone, a tablet computer and a laptop computer. It is also possible to combine a control unit with integrated control elements and a remote control, whereby the user may choose whether to use the integrated control unit or the remote control, or both, for controlling the irrigation process.

The wireless communication between the control unit and a remote control or a remote unit may be obtained in many ways, as is per se well known in the art, such as by infrared light (IR), ultrasonic communication, radio frequency (RF) communication such as Bluetooth, etc.

The control unit is further preferably provided with a battery for driving the controller, the electrically operated pump(s) and the electrically operated valve.

The controller is preferably programmable. For example, the controller may be programmable to set the desired flow rate. Further, the controller may be programmable to set total irrigation liquid volume to be discharged in addition to or instead of setting a desired flow rate. The controller may be pre- programmed with a number of programs, or it may be programmed via the control element(s) or through an external remote control or the like. The controller may be programmable so as to automatically carry out a predefined program. A user that frequently uses anal irrigation may experience a preferred way of carrying out the irrigation process. Then it is of advantage to be able to program this way into the controller, so that the irrigation process is done in the most preferred way every time. Furthermore, caregivers may have a certain experience concerning the optimum process, which they can program into the controller. Thereby errors will be reduced.

Further, the controller may be programmable to set a maximum filling level of the inflatable retention member. The maximum filling level may be a fixed level, defined by the producer, a physician or the like, or a user defined, customizable level, determined by the user, or a combination of both. This increases the safety of the irrigation system, since inadvertent overfilling of the inflatable retention member can hereby be avoided.

Preferably, all components of the irrigation system are individually exchangeable, so that e.g. the probe/catheter can be exchanged frequently, and typically be used only once, whereas other parts of the system, such as the control unit, the electrical system and the irrigation liquid reservoir can be used for months or even years.

The irrigation system of the present invention comprises relatively few and uncomplicated components, and which may be reused for a long time, which makes the irrigation system relatively easy and cost-efficient to produce. Further, the irrigation system lends itself well for automated or semi-automated manufacturing.

The irrigation system of the present invention is also highly suitable for self-administration of the irrigation liquid. The control elements on the control unit also make it easy to access the pump with one hand only, and to switch between different pumping modes etc. Typically with this arrangement, it is e.g. possible to operate the irrigation system with one finger, e.g. the thumb. This provides a very convenient and precise controllability of the irrigation system.

The control of the present invention is also relatively simple to realize, install and operate, thereby making the resulting method/system cost-efficient and user friendly.

The outflow rate and volume will also vary in dependence of the temperature. However, since the irrigation system will normally be used at room temperature the temperature dependent variation will for all practical use situations be neglectable. However, for rare situations where the temperature has a significant variation, or if there is a need to improve accuracy even further, the irrigation system may also comprise a temperature sensor. In this case, the temperature sensor is preferably arranged on or in the reservoir, thereby to sense and determine the temperature on or in the reservoir. To further increase accuracy, a temperature sensor may also be arranged to determine the ambient temperature outside the reservoir.

For improved accuracy, the system may also further comprise a pressure sensor to determine the ambient pressure.

These and other aspects of the invention will be apparent from, and elucidated with, reference to the embodiments described hereinafter.

### BRIEF DESCRIPTION OF THE DRAWINGS

For exemplifying purposes, the invention will be described in closer detail in the following with reference to embodiments thereof illustrated in the attached drawings, wherein:
Fig. 1 illustrates an irrigation system in accordance with an embodiment of the present disclosure;
Fig. 2 schematically illustrates a side view of a container useable in embodiments of the present disclosure;
Fig. 3 schematically illustrates a side view of a container useable in another embodiment of the present disclosure;
Fig. 4 is a schematic block diagram of an irrigation system in accordance with an embodiment of the present disclosure;
Fig. 5 is a schematic block diagram illustrating the parts the irrigation system in Fig. 4, useable for irrigation fluid flow control in more detail; and
Fig. 6 is a schematic block diagram illustrating a possible realization of the flow control system of Fig. 5 in more detail.

### DETAILED DESCRIPTION OF CURRENTLY PREFERRED EMBODIMENTS

The irrigation system of the present disclosure is particularly useful for rectal and transanal irrigation. However, the irrigation system may also be used in other types of medical irrigation procedures. The following discussion is, in particular, concerned with the preferred field of use, rectal and transanal irrigation systems, even though the disclosure is not limited to this particular type of irrigation systems.

It is to be understood that features of the different embodiments are exchangeable between the embodiments and may be combined in different ways, unless anything else is specifically indicated. It may also be noted that, for the sake of clarity, the dimensions of certain components illustrated in the drawings may differ from the corresponding dimensions in real-life implementations of the invention, e.g. the length and width of the reservoir/container, etc.

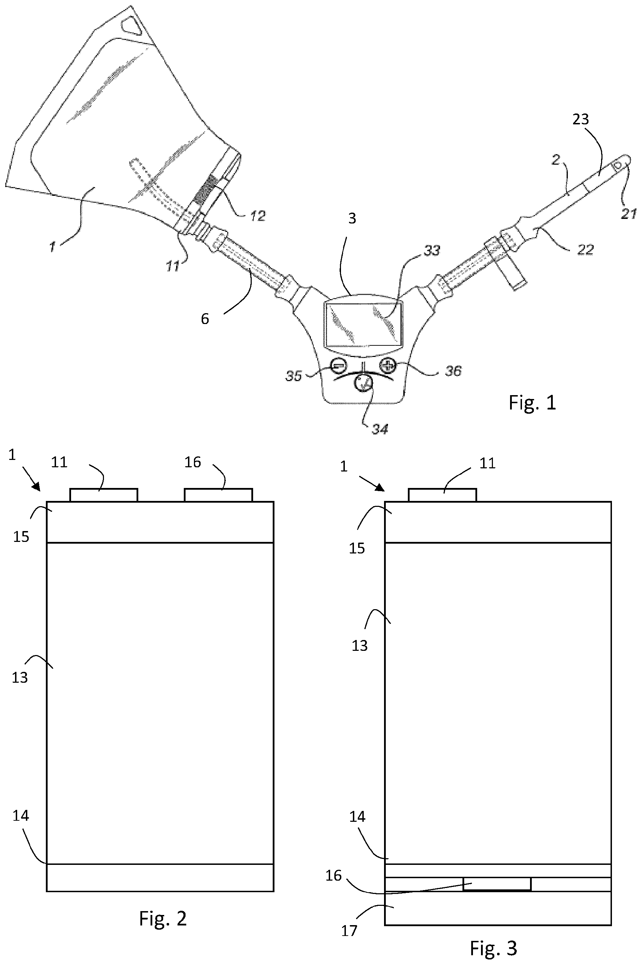

Fig. 1 discloses an irrigation system according to a first exemplary embodiment, comprising a container 1 for an irrigation liquid, a catheter or probe 2 for arrangement in a user, and a control unit 3.

The container may be realized in various ways. For example, the container may be formed by a rigid, semi-rigid or flexible material. In case a semi-rigid or flexible material is used, the container may be collapsible or foldable, to make the irrigation system more compact prior to use. The container is preferably provided with an opening, closed by a closure 11, for filling of the reservoir. Tubing connecting the container to the rest of the irrigation system may be provided through the closure 11, or through other access points on the container.

In order to render the irrigation system as portable as possible, the container preferably has a capacity of less than 5 liters, more preferred less than 3 liters and most preferred less than 2 liters. If, however, the system is to be used for repeated irrigation, a larger capacity container may be necessary.

The container may comprise an overpressure release valve, to release pressure over a predetermined maximum pressure to be allowed. Further, the container may also, optionally, comprise a filter 12, such as a hydrophobic filter, which is impermeable to the irrigation liquid, but which allows air to enter the reservoir but not escape the container. Such a filter ensures that the container maintains its shape when irrigation liquid is being pumped out from the container.

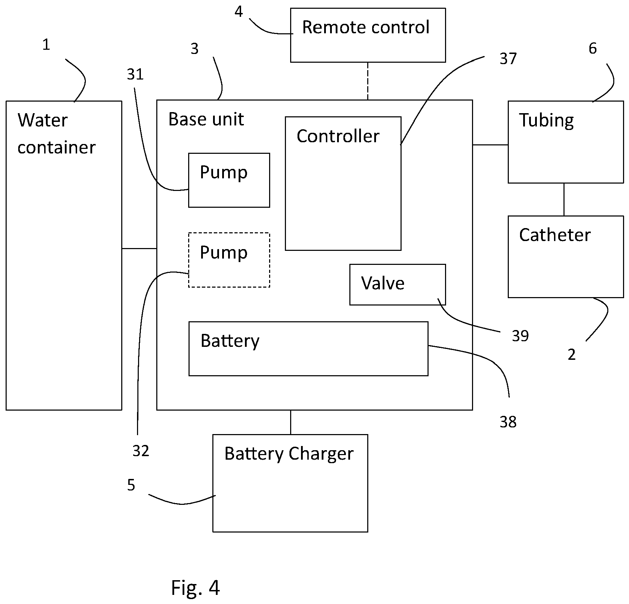

A pump 31, best seen in Fig. 4, may be used to pump air into the container for, indirectly, pumping liquid out of the container.

The catheter/probe 2 is provided with a retention member, such as an inflatable balloon 23, in the vicinity of an insertable end 21, for fixing the catheter in a body cavity.

The pump 31 used for indirect pumping of irrigation liquid from the water container may also be used for pumping air or other fluids into the inflatable retention element. The pump 31 may e.g. be arranged to pump air, and valves may be used to direct the pumped air into the water container and the inflatable retention member, respectively. However, alternatively, a second pump 32 may be provided to pump air or other fluids into the inflatable retention member.

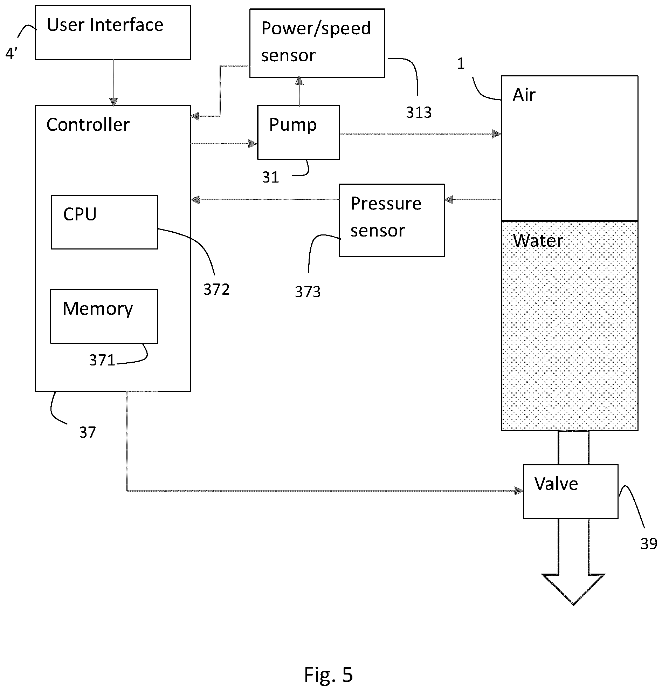

The pump 31 is arranged for indirect pumping of the irrigation liquid. Hereby, the pump pumps a fluid, preferably a gas such as air, through a conduit to the reservoir. Thereby, pressure increases in the reservoir to pump irrigation liquid out from the reservoir through another conduit. This conduit preferably passes through an electrically operable valve 39, best seen in Fig. 5, and continues to the probe, for dispensing the irrigation liquid to the user. The valve 39 is preferably connected to the controller 37 (see Fig. 4), so that the controller may control the degree of openness of the valve. In case a flow sensor is provided, the input from the flow sensor may be used by the controller to regulate the valve.

The valve 39 may be an on/off valve, arranged only to assume a fully opened or fully closed state. However, the valve may also provide intermediate positions, and may e.g. be gradually controllable between these end states. Such an electrically operable valve can be realized in many ways, as is per se well-known in the art. For example, the electrically operable valve may be a clamp or pinch valve, providing a controllable clamping/pinching action on a tube leading between the electrical pump and the probe. For example, the valve may be of the type disclosed in US 2006/0114148 by the same applicant, said document hereby being incorporated in its entirety by reference.

The one or more pumps 31, 32 are preferably electrically operated pump(s). The pumps may e.g. be diaphragm pumps, which are generally non-reversible, but other pump types, such as peristaltic pumps, which may be reversible, are also feasible.

The reservoir/container 1 may be realized in various ways. For example, the reservoir may be formed by a rigid, semi-rigid or flexible material. In case a semi-rigid or flexible material is used, the reservoir may be collapsible or foldable, to make the irrigation system more compact prior to use. The reservoir is provided with an opening, closed by a lid 11, for filling the reservoir. Tubing 6, connecting the reservoir to the rest of the irrigation system, may be provided through the lid 11 or through other access points on the reservoir.

The catheter 2 is here embodied as a rectal catheter. The catheter is provided with an inflatable retention member 23, such as an inflatable balloon, for fixing the catheter in a body cavity. The inflatable balloon preferably protrudes as a toroidal shape when inflated and is essentially flush against the wall of the catheter when deflated. The inflatable retention member may also be referred to as a balloon, and is arranged close to the insertable tip 21, but at some distance from the end. Between the tip and the balloon, an opening for dispensing liquid such as irrigation liquid to, or draining liquid from, the body may be provided. The inflatable retention member may be made of any suitable material, such as PVC, latex, TPE or PU. However, other materials providing similar properties can likewise be used.

Further, the probe may be provided with a rearward enlarged part 22, providing an abutment to hinder too deep insertion. The catheter/probe is preferably provided with two lumens - one lumen for transfer of irrigation liquid through the probe, for discharge at the forward end, and one lumen for inflation and deflation of the balloon.

The probe/catheter may be of the type disclosed in WO 2014/154635, said document hereby being incorporated in its entirety by reference.

A control unit 3 may be provided. The control unit may be realized as a unitary, handheld unit, as in the illustrative example of Fig. 1. However, the control unit may alternatively be arranged as a base unit, e.g. arranged to be placed on the ground or floor during use.

As one embodiment, the reservoir/container may be a collapsible reservoir of the type disclosed in US 2015/0335529, said document hereby being incorporated in its entirety by reference.

In order to render the irrigation system as portable as possible, the container preferably has a capacity of less than 5 liters, more preferred less than 3 liters and most preferred less than 2 liters. However, when the system is to be used for repeated irrigation, a larger capacity container may be necessary.

The irrigation liquid can be any liquid which is capable of irrigating the body cavity of interest. In order to stimulate bowel movements, suitable irrigation liquids include water, hypertonic aqueous salt solutions, solutions or suspensions of cathartic agents, such as bisacodyl or phenolphthalein, and mineral oil.

Referring to Figs. 2 and 3, the container 1 may comprise a flexible side wall member 13, a rigid bottom portion 14, a rigid top portion 15, an inlet opening 11, and an outlet opening 16.

In the embodiments illustrated in Figs. 2 and 3, when assembled, the bottom portion 14 is arranged at a first open end of the side wall member 13 such that it seals the first open end. The rigid top portion 15 is arranged at a second open end of the side wall member 13 such that it seals the second open end.

In the embodiment of Fig. 2, both a filling opening 11 and an outlet opening 16 are provided in the rigid top portion. However, alternatively, as illustrated in Fig. 3, the outlet opening may instead be provided in the rigid bottom portion 14, which may e.g. be docked into a base portion 17. In such embodiments, the base portion 17 may comprise the controller 3, then realized as a base unit. The side wall 13 may be made from a flexible sheet material.

The control unit may be arranged connected to the container and the probe through tubing. Alternatively, the control unit may be directly connected to, and possibly even integrated with, the irrigation liquid container.

The first electrically operated pump 31, as well as the optional second electrically operated pump 32, for pumping irrigation liquid and inflation fluid may be provided within the control unit 3, as the illustrative example of Figs. 4 and 5, but may also be arranged outside the bounds and housing of the control unit.

The control unit may comprise a display 33, and a user interface for providing input to the system, e.g. including one or several control elements. In the example of fig. 1, three control elements 34, 35 and 36 are provided. The control elements are preferably realized as depressible control buttons. The control unit is preferably waterproof. The control elements may thus be realized with thick pliable plastic or the like, designed to withstand many pushes.

However, alternatively, as in the illustrative example of Fig. 4, the control elements may instead be provided on a remote control 4, and may e.g. be realized as sensor areas on a touch screen.

The control elements 35 and 36 may here be used to activate the pump(s) for inflation/deflation of the inflatable retention member, transfer of irrigation liquid through the probe for irrigation (control element 36), releasing overpressure and/or draining the system of remaining liquid (control element 35). Separate control elements may also be provided for irrigation and inflation, so that inflation and deflation of the retention member may take place independently of the irrigation, and e.g. simultaneously.

The control unit or base unit comprises a controller 37. The control unit or base unit may further comprise one or more of the above-discussed pumps 31, 32 and a battery 38.

The controller 37 may be a microprocessor, MCU, comprising one or several central processing units, CPUs. The controller may also comprise two or more MCUs. However, the controller may also be realized in other ways, as is per se known in the art.

The irrigation system may further comprise a memory, , either arranged integrated with the controller 37, or arranged as a separate component connected to the controller. The memory may be storing a set of inflation levels, each level being correlated to a volume, and a user interface 4' (see Fig. 5) arranged to receive input of a selected inflation level selected form said set of inflation levels. The controller 37 may then be arranged to operate the electrically operated pump until a desired and selected irrigation volume has been reached. To this end, the controller 37 is hereby arranged to monitor the present fluid rate volume over time to determine an aggregated volume that has been irrigated, and abort pumping as soon as the desired volume level has been reached. The memory may be a ROM memory, such as an EPROM or EEPROM, or a RAM memory, such as Flash memory. However, many other types of memories may also be used, as is per se well known in the art.

Further, the controller is optionally connected to a wireless transceiver, which is adapted to transmit and receive data from the remote control 4, and/or other remote units. Hereby, the remote control may provide control data to the controller 37, for remote control of the control unit. Additionally or alternatively, the controller may transmit data about the irrigation procedure to the remote control or any other remote unit. The remote control 4 may e.g. be a smart phone or the like.

The battery 38 may be a rechargeable battery, chargeable through an external battery charger 5. Charging may e.g. occur through inductive charging from the charging station 5, or as direct charging from a connected electric conductor.

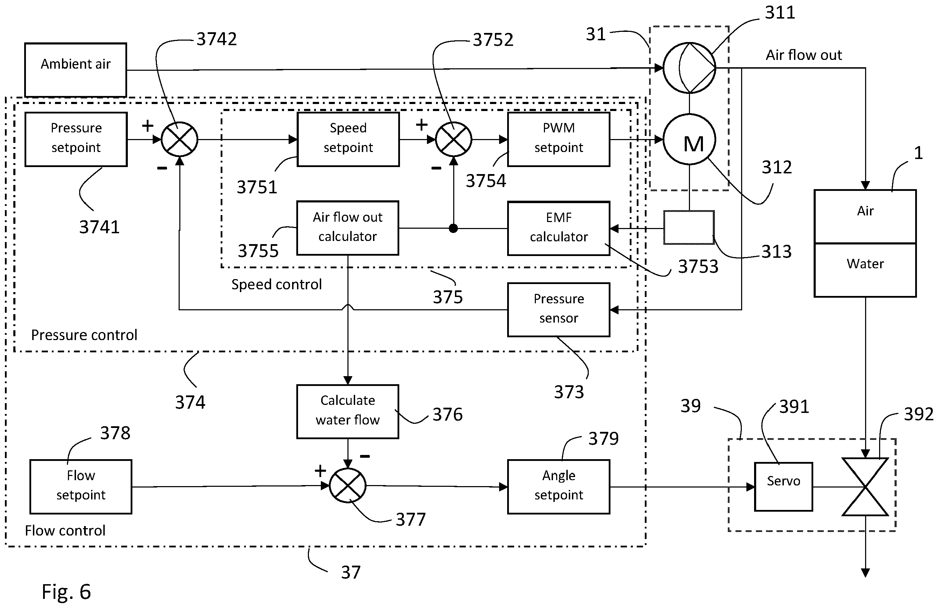

The part of the system handling the irrigation liquid discharge will now be discussed in more detail with reference to Figs. 5 and 6.

With reference to Fig. 5, a pump 31 is arranged to pump air through a conduit to the reservoir 1. Due to the air pumped into the reservoir, the pressure in the reservoir increases, thereby creating a flow of water out from the reservoir.

The conduit leading water out from the reservoir 1 passes an electrically operable valve 39.

A pressure sensor 373 is arranged to determine the pressure in the reservoir 1. Output from the pressure sensor is sent to the controller 37. The controller 37 may, as discussed in the foregoing, comprise a processor, such as a CPU 372, and a memory 371. The controller 37 communicates with a user interface 4' to receive input regarding e.g. a desired flow rate.

The pressure sensor may be any type of per se known pressure sensors. Preferably, the pressure sensor is a gauge pressure sensor, measuring the pressure relative to atmospheric pressure, and may e.g. be a piezoresistive sensor, such as a piezoresistive strain gauge, a capacitive sensor, an electromagnetic sensor, an optical sensor or the like. However, other types of pressure sensors may also be used. The pressure in the inflatable retention member can be directly determined by measuring by a pressure sensor. However, the pressure in the inflatable retention member may also be indirectly measured. For example, the pressure sensor may be a sensor measuring the load on the pump, from which it is possible to estimate the pressure in the reservoir.

The controller 37 controls the electrically operated pump 31 and the electrically operated valve 39, based on the determined pressure in the reservoir, as provided by the pressure sensor 373, a determined power consumption and/or speed of the pump, as provided by a power and/or speed sensor 313, and the desired flow rate, as received form the user interface 4'. The power sensor may e.g. be a current and/or voltage sensor, determining the current and/or voltage provided to the pump. The speed sensor may e.g. be a tachometer.

The controller 37 may be arranged to operate the pump so that an essentially constant pressure is provided and maintained in the reservoir. However, in other embodiments, the pump may instead be operated to provide a varying pressure in the reservoir. For such embodiments, the system may further be provided with a water level sensor or the like, arranged to provide an estimate of the current volume of water in the reservoir to the controller.

The controller 37 is arranged to estimate an actual flow rate of irrigation liquid from the reservoir to the catheter based on at least the estimation of the power consumption and/or speed and to continuously regulate the electrically operable valve based on the estimated actual flow rate, thereby adjusting the actual flow rate to the desired flow rate.

A more detailed exemplary realization of the system of Fig. 5 will now be discussed with reference to Fig. 6.

As illustrated in Fig. 6, the controller may comprise a pressure control 374, which in turn may comprise a speed control 375.

In the pressure control 374 the pressure in the reservoir 1 is determined by the pressure sensor 373, and this determined pressure is used in conjunction with a desired pressure, a pressure setpoint 3741, in regulation unit 3742 to provide a pressure control signal to control the operation of the pump 31 through the speed control 375. In this embodiment, the electrically operated pump comprises a motor 312 driving a mechanical pump 311. The pressure level determined by the pressure setpoint may be a fixed pressure level, which may be a predetermined level, for embodiments where the system is arranged to maintain a constant pressure in the reservoir.

The speed control 375 receives the pressure control signal and determines a corresponding desired speed for the pump motor 312 in a speed setpoint 3751. The desired speed is forwarded to a regulation unit 3752.

One or more power consumption and/or speed sensor 313 may be used to determine the power consumption and/or speed of the motor. The pump motor may e.g. be a brushed DC motor, and the power consumption sensor may e.g. comprise a voltage difference sensor and a current sensor.

The output from the sensor(s) 313 may be forwarded to an EMF calculator 3753, estimating an electromagnetic force (EMF) and/or speed of the pump motor 312. The output from the EMF calculator 3753 may also be forwarded to the regulation unit 3752.

The regulation unit 3752 may use the desired speed from the speed setpoint 3751 in conjunction with the estimated power consumption and/or speed from the EMF calculator 3753 to determine speed control signal. This signal is forwarded to a PWM setpoint 3754, determining a pulse width modulation (PWM) for driving the pump motor 312.

The power consumption and/or speed signal provided by the EMF calculator 3753 is further forwarded to an air flow out calculator 3755, which uses this to provide an estimate of the flow rate and/or volume of air pumped by the electrically operated pump 31.

The estimate provided by the air flow out calculator 3755 is forwarded to a unit 376 calculating a nominal output flow rate of water from the reservoir based on this estimated pumped air flow and/or volume. The nominal output flow rate of water corresponds to a flow rate that will occur when the valve is in a fully opened state, i.e. a maximum output flow rate, where there are no restrictions in the water outflow.

A further unit, a flow setpoint 378, determines a desired flow rate, e.g. based on input from a user interface. The desired flow rate and the estimated nominal output flow rate are used in conjunction by regulation unit 377 to provide a valve control signal.

As discussed in the foregoing, the valve may be of the type disclosed in US 2006/0114148. The electrically operable valve may comprise a movable arm that is connected to a constriction structure, said constriction structure being arranged opposite to an abutment, and with the tube forwarding the irrigation liquid from the reservoir to the catheter extending between the constriction structure and the abutment. Movement of the moveable arm moves the constriction structure to control compression of the tube between the constriction structure and the abutment. The valve can be controlled by the controller 37 to assume a fully opened state and a fully closed state, and preferably also intermediate states. In an embodiment, the valve is continuously controllable between any state between the fully closed and opened states. The mechanical valve 392 may comprise a moveable arm which may be movable by rotation, and the rotation may be effected by a servo motor 391. The angular position of the moveable arm will consequently determine the degree of openness of the valve.

In the illustrative example, the valve control signal from the regulation unit 377 may be forwarded to an angle setpoint 379, where an angle for the moveable arm in the valve 39 is determined based on the valve control signal and forwarded to the servo 391 to move the movable arm into this angular position.

In use, the reservoir is filled with a desired volume of irrigation liquid. The pump is then operated to prime the tank and increase the pressure to a target level. The system is then used for irrigation. Hereby, the pump may be continuously operated to maintain the pressure in the reservoir constant, when the electrically operated valve is opened to release irrigation liquid out from the reservoir. The flow rate of the irrigation liquid out from the reservoir is estimated based on the power consumption and/or speed of the pump, and is used to control the electrically operated valve to obtain a desired output flow rate.

The person skilled in the art realizes that the present invention by no means is limited to the preferred embodiments described above. On the contrary, many modifications and variations are possible within the scope of the appended claims. For example, the pump can be operated to maintain an essentially constant pressure in the reservoir, regardless of the volume of irrigation liquid. However, alternatively, the pressure in the reservoir may be allowed to vary over time. Further, the irrigation liquid may be water, but also other irrigation liquids are feasible. The gas pumped into the reservoir is preferably air, but other gases may also be used. Further, one or more sensors used to determine the power consumption and/or speed of the pump may be the only sensors used for determination of the flow rate of irrigation liquid out from the container. This is e.g. feasible when the pressure sensor is used solely to maintain a constant pressure in the reservoir. Alternatively, the one or more sensors used to determine the power consumption and/or speed of the pump and the pressure sensor may be the only sensors used for determination of the flow rate of irrigation liquid out from the container. The irrigation system could also be used for other purposes than rectal or transanal irrigation, such as other forms of irrigation, or for other medical procedures.

Such and other obvious modifications must be considered to be within the scope of the present invention, as it is defined by the appended claims. It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting to the claim. The word "comprising" does not exclude the presence of other elements or steps than those listed in the claim. The word "a" or "an" preceding an element does not exclude the presence of a plurality of such elements. Further, a single unit may perform the functions of several means recited in the claims.

## Claims

1. An irrigation system comprising:
a reservoir for an irrigating liquid;
a pressure sensor for measuring the pressure in the reservoir;
a catheter for arrangement in a user;
tubing providing a fluid communication path between said reservoir and said catheter;
an electrically operated pump for pumping air into the reservoir, thereby to indirectly pump irrigation liquid from the reservoir to the catheter through said tubing;
an electrically operable valve operable to continuously control the degree of openness of said fluid communication path between a fully closed state and a fully opened state;
a sensor for estimation of power consumption or speed of the electrically operated pump;
a controller arranged to estimate an actual flow rate of irrigation liquid from the reservoir to the catheter based on said estimation of the power consumption and/or speed, to obtain a desired flow rate from a user interface, and to continuously regulate the electrically operable valve based on the estimated actual flow rate, thereby adjusting the actual flow rate to said desired flow rate.

2. The irrigation system of claim 1, wherein the controller is further arranged to control a pumping speed of the electrically operated pump based on the measured pressure in the reservoir to provide an essentially constant pressure in the reservoir over a range of irrigation liquid filling levels.

3. The irrigation system of claim 2, wherein the controller is arranged to control the pumping speed of the electrically operated pump by controlling or measuring the voltage and/or current supplied to the electrically operated pump.

4. The irrigation system of any one of the preceding claims, further comprising a water level sensor for determining a current irrigation liquid level in said reservoir.

5. The irrigation system of any one of the preceding claims, wherein the electrically operated pump is at least one of a peristaltic pump and a diaphragm pump.

6. The irrigation system of any one of the preceding claims, wherein the electrically operable valve is a clamping or pinch valve, providing a controllable clamping/pinching action on the tubing.

7. The irrigation system of claim 6, wherein the electrically operable valve comprises a movable arm that is connected to a constriction structure, said constriction structure being arranged opposite to an abutment, and with the tubing extending between said constriction structure and said abutment, whereby movement of the moveable arm moves the constriction structure to control compression of the tubing between said constriction structure and said abutment.

8. The irrigation system of claim 7, wherein the moveable arm is rotatable, and comprising a cam shape engaging said constriction structure.

9. The irrigation system of any one of the preceding claims, wherein the catheter is provided with an inflatable retention member.

10. The irrigation system of claim 9, wherein the electrically operated pump is operable also for inflation of the inflatable retention member.

11. The irrigation system of any one of the preceding claims, further comprising a control unit with a housing, said housing enclosing said electrically operated pump, said electrically operable valve and said controller.

12. The irrigation system of any one of the preceding claims, wherein the electrically operable valve is operable to continuously control the degree of openness of said fluid communication path between a fully closed state and a fully opened state over a plurality of intermediate states between said fully closed and fully opened state, and preferably over essentially all possible intermediate states.

13. The irrigation system of any one of the preceding claims, wherein the sensor for estimation of power consumption or speed of the electrically operated pump comprises a current and/or voltage sensor.

14. The irrigation system of any one of the preceding claims, wherein the sensor for estimation of power consumption or speed of the electrically operated pump comprises a speed sensor, and preferably a tachometer.
